# EUROPEAN PATENT SPECIFICATION

(11) **EP 2 656 387 B1**
(45) Date of publication and mention of the grant of the patent: **30.07.2014**
(21) Application number: 11824335.1
(22) Date of filing: 19.12.2011
(51) Int. Cl.: H01L 27/082, H03F 3/343, H03K 17/04, H01L 29/10, H01L 29/66

(54) **COMPLEMENTARY DARLINGTON EMITTER FOLLOWER WITH IMPROVED SWITCHING SPEED AND IMPROVED CROSS-OVER CONTROL AND INCREASED OUTPUT VOLTAGE**
KOMPLEMENTÄRER DARLINGTON-EMITTERFOLGER MIT VERBESSERTER SCHALTGESCHWINDIGKEIT UND VERBESSERTER KREUZUNGSSTEUERUNG UND ERHÖHTER AUSGANGSSPANNUNG
ÉMETTEUR ASSERVI COMPLÉMENTAIRE DARLINGTON À VITESSE DE COMMUTATION AMÉLIORÉE ET À RÉGLAGE DE CROISEMENT AMÉLIORÉ, ET À TENSION DE SORTIE ACCRUE

(30) Priority: 20.12.2010 US 201061424956 P
(43) Date of publication of application: 30.10.2013
(73) Proprietor: Diodes Zetex Semiconductors Limited, Chadderton Oldham OL9 9LL (GB)
(72) Inventor: CASEY, David, Neil, Ramsbottom Lancs BL0 0ES (GB)
(74) Representative: Zahn, Matthias
(86) International application number: PCT/IB2011/003282
(87) International publication number: WO 2012/085677

(56) References cited:
- EP-A1- 0 366 213
- EP-A2- 0 827 271
- WO-A1-89/06070
- GB-A- 2 239 750
- US-A- 3 769 568
- US-A- 4 404 478
- US-A- 4 510 677
- US-A- 5 057 714

## Description

### BACKGROUND

Particular embodiments generally relate to Darlington transistor configurations.

Use of a complementary darlington pair in an emitter follower configuration has a number of known limitations. Specifically, charge stored in the base of the output transistor restricts switching speed due to the fact that there is no low impedance route by which the charge can be quickly removed. Also, the bases of the two output transistors are not connected by a low impedance link, which means that at the time when one transistors base is changing state from an "OFF" condition to an "ON" condition, the base of the complementary output transistor is not guaranteed to be, and indeed will not be at the same potential, i.e., will not be simultaneously changing from its previously existing "ON" condition to an "OFF" condition. Thus, both transistors are turned on during the cross-over period. This results in a high unwanted "through" conduction current flowing from supply to ground, degrading efficiency. Also, the maximum output voltage swing cannot closely approach that of a single transistor complementary pair because each darlington cannot have less then VBE(∼0.9V) plus VCESAT (∼0.2V) across it's collector emitter when turned on.

Document EP 0827271 discloses an output or final stage of a power amplifier which uses a complementary pair of npn and pnp power transistor SEPP (single ended push-pull) connected to output a large power.

Document US 3 769 568 discloses a dc-to-dc converter having soft start and other regulation features employing priority of pulse feedback.

Document JP 11097679 discloses a Darlington coupled main I6BT and an auxiliary IGBT.

Document US 4028561 discloses a fast switching Darlington circuit providing a completely integrated npn Darlington stage.

In one embodiment, a circuit is disclosed as recited in claim 1.

The following detailed description and accompanying drawings provide a better understanding of the nature and advantages of the present invention.

### BRIEF DESCRIPTION OF THE DRAWINGS

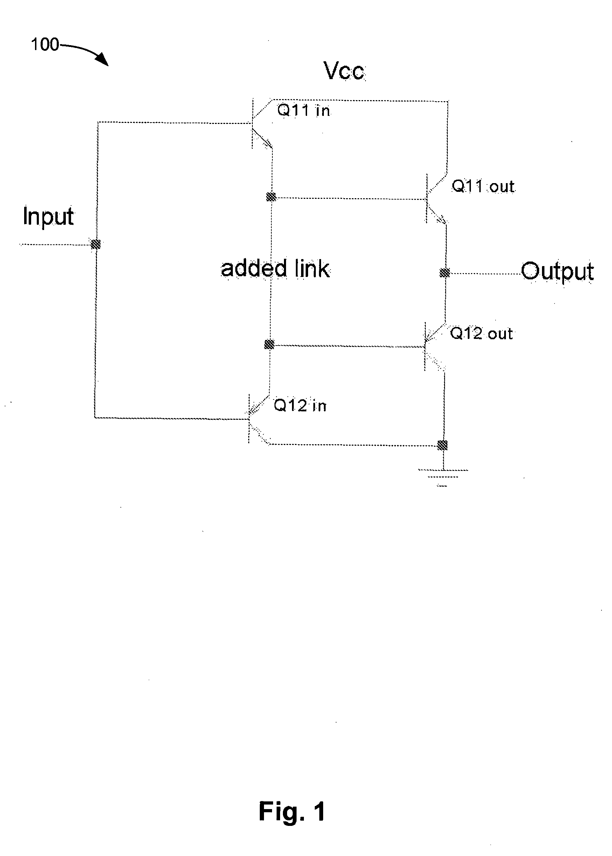

Fig. 1 depicts a circuit of a Darlington transistor configuration according to one embodiment.

### DETAILED DESCRIPTION

Described herein are techniques for a Darlington transistor configuration. In the following description, for purposes of explanation, numerous examples and specific details are set forth in order to provide a thorough understanding of embodiments of the present invention. Particular embodiments as defined by the claims may include some or all of the features in these examples alone or in combination with other features described below, and may further include modifications and equivalents of the features and concepts described herein.

Fig. 1 depicts a circuit 100 of a Darlington transistor configuration according to one embodiment. In one embodiment, the Darlington transistor configuration is a complementary Darlington emitter follower pair. Circuit 100 may be used to drive MOSFET and Insulated gate bipolar transistor (IGBT) gates with a high current gain. As shown, circuit 100 includes a first transistor Q11 IN and a second transistor Q11 OUT that may be considered a first Darlington. A complementary Darlington or emitter follower may include transistor Q12 IN and transistor Q12 OUT. In one embodiment, circuit 100 may be a monolithic Darlington configuration. The monolithic configuration means that transistor Q11 IN and Q11 OUT may be included in a single die and transistor Q12 IN and transistor Q12 OUT may be included in a single die. Both dies may be included in a single device. Also, it will be understood that even though a Darlington configuration is described, other similar configurations may be used, such as a triplington or other multiple transistor configurations in a Darlington configuration. Also, NPN and PNP variations of the Darlington transistor configuration may vary.

In circuit 100, an input is coupled to the base of transistor Q11 IN and the base of transistor Q12 IN. Also, an output is coupled to the emitters of transistor Q11 OUT and transistor Q12 OUT. The emitters of transistors Q11 OUT and Q12 OUT may be connected together (shorted) or left unconnected in which case there are two output nodes. Particular embodiments add a link between the base of transistor Q11 OUT and the base of transistor Q12 OUT. The added link may be a wire that provides a short. Adding the link eliminates or reduces a very large wasted shoot-through current at a crossover. At a point in the switching cycle where transistor's Q11 OUT state is being changed from conducting to non-conducting and transistor Q12 OUT is changing from non conducting to conducting, transistor Q11 OUT should turn off at exactly the same time that transistor Q12 OUT turns on. However, due to charge stored in the base of transistor Q11 OUT, transistor Q11 OUT remains turned "ON" for a finite time during which time transistor Q12 OUT and transistor Q11 OUT are both ON simultaneously and present a very low resistance path to ground. During this time, very large shoot through currents flow from supply to ground. These currents are wasted energy and reduce the efficiency of the circuit. The provision of the short from the base of transistor Q11 OUT to the base of Q12 OUT resists the tendency for both transistors to be simultaneously turned on. Also, it provides a very effective route for removal of the stored charge and thus further resists the possibility of transistor Q11 OUT remaining turned on for long enough to permit the flow of the shoot through currents. An identical situation exists when transistor Q12 OUT is turning off and transistor Q11 OUT is turning on.

Also, circuit 100 provides a highest possible switching speed and the highest possible rail to rail excursion. The highest switch speed is due to being able to discharge transistor Q11 OUT or transistor Q 12 OUT quickly with the added link. In addition, a turn-off voltage is lowered to about 0.5V and also the turn-on voltage is within 0.5V of supply Vcc. Conventionally, the turn off voltage or turn on voltage would not be lower than one VBE + one VCESAT ∼ 0.9V, where VBE is the base-emitter voltage and VCESAT is the collector-emitter voltage at saturation. This is because the base of the output transistor Q11 OUT or transistor Q12 OUT is required to be at approximately 0.7V above its emitter voltage for base current to flow to create current flow in its emitter-collector circuit. Additionally this base current is supplied by the input transistor Q12 IN via its emitter-collector circuit. In a monolithic, or discrete darlington configuration, the collector of the input transistor Q12 IN is connected to the collector of the output transistor Q12 OUT. Hence the voltage difference between the emitter of transistor Q12 IN (which is also the base of transistor Q12 OUT) and its collector cannot be less than the VCESAT of transistor Q12 IN, say 0.2V, otherwise transistor Q12 IN will not conduct the needed base current into the base of transistor Q12 OUT. Given that the emitter of transistor Q12 OUT has to be approximately 0.7V below its base and its base has to be 0.2V below its collector then the collector emitter voltage of transistor Q12 OUT when conducting cannot be less than approximately 0.7V plus 0.2V, that is - VBESAT(of transistor Q12 OUT) plus VCESAT(of transistor Q12 IN).

A wire may be used to couple the base of transistor Q11 OUT and the base of transistor Q12 OUT together. The wire permits the characteristics of the Darlingtons to be modified as desired. Additional components other than the wire may be added with the wire or used in lieu of the wire.

Also, an additional pad may be provided such that the wire may be added to link transistor Q11 OUT and transistor Q12 OUT when a monolithic darlington design is used. The additional pad may not be necessary if a monolithic darlingon design is used. The Darlingtons may be cascaded where additional pads may or may not be provided such that each intermediate base may or may not be connected to an additional component.

Without using the added link, it is possible that transistors Q11 OUT and Q12 OUT were on at the same time. This is because the bases of transistor Q11 OUT and transistor Q12 OUT do not have a low resistance route by which charge stored in their bases can be removed rapidly. Whichever transistor is turned "ON" contains significant stored charge that must be removed before its voltages will start to change. If the base bias difference reaches around 1.2V, both transistors Q11 OUT and Q12 OUT are on. This causes a current to flow from power supply Vcc to ground through transistors Q11 OUT and Q 12 OUT. This is a high parasitic current, which wastes power.

The link causes the bases of transistors Q11 OUT and Q 12 OUT to be at the same voltage and provides a route for the rapid removal of the stored base charge. Thus, transistor Q11 OUT and transistor Q12 OUT do not turn on at the same time. Thus, a very large parasitic current does not flow from power supply Vcc to ground.

The added link also allows the switching performance of the Darlington transistor to be fast. The switching performance may be dependent upon charge stored in the base region of transistor Q11 OUT. If the base region of transistor Q11 OUT is floating, then the time in which the charge can dissipate may limit switching times to turn off transistor Q11 OUT from an on state. However, the added link allows charge to be dissipated faster from the base region to turn off transistor Q11 OUT. For example, transistor Q12 IN turns on, and couples the base of transistor Q11 OUT to ground. This dissipates the charge at the base of transistor Q11 OUT to decrease the switching time to turn off transistor Q11 OUT. The discharge is performed for transistor Q12 OUT through transistor Q11 IN but in the opposite direction.

As used in the description herein and throughout the claims that follow, "a", "an", and "the" includes plural references unless the context clearly dictates otherwise. Also, as used in the description herein and throughout the claims that follow, the meaning of "in" includes "in" and "on" unless the context clearly dictates otherwise.

The above description illustrates various embodiments of the present invention along with examples of how aspects of the present invention may be implemented. The above examples and embodiments should not be deemed to be the only embodiments, and are presented to illustrate the flexibility and advantages of the present invention as defined by the following claims. Based on the above disclosure and the following claims, other arrangements, embodiments, implementations and equivalents may be employed without departing from the scope of the invention as defined by the claims.

## Claims

1. A circuit (100) comprising:
a Darlington transistor (Q11 IN, Q11 OUT), which includes:
a first input transistor (Q 11 IN) having a base connected to an input (Input);
a first output transistor (Q11 OUT) having a base connected to the emitter of the first
input transistor (Q 11 IN); and
a further transistor (Q12 IN) complementary to the first input transistor (Q 11 IN) **characterized by**
having a base shorted to the base of the first input transistor (Q11 IN), and an emitter shorted to the base of the first output transistor (Q11 OUT).

2. The circuit (100) of claim 1, in which the further transistor (Q 12 IN) is of a type complementary to the first output transistor (Q11 OUT) of the Darlington transistor (Q11 IN, Q11 OUT).

3. The circuit (100) of claim 1, in which the further transistor (Q 12 IN) is an input transistor (Q 12 IN) of a further Darlington transistor (Q 12 IN, Q 12 OUT).

4. The circuit (100) of claim 3, in which the further Darlington transistor (Q 12 IN, Q 12 OUT) includes a second output transistor (Q 12 OUT) having an emitter connected to an emitter of the first output transistor (Q 11 OUT).

5. The circuit (100) of claim 1, in which the further transistor (Q 12 IN) is connected to ground.

6. The circuit (100) of claim 1, in which the further transistor (Q 12 IN) is connected to a supply (Vcc).

7. The circuit (100) of claim 1, in which the first input transistor (Q 11 IN) has a collector connected to the supply (Vcc) and the further transistor (Q12 IN) has a collector connected to ground.

8. The circuit (100) of claim 1, wherein the base of the further transistor (Q 12 IN) is shorted to the base of the first input transistor (Q 11 N) by a shorting link (added link) that removes charge stored in the base of the first input transistor (Q 11 N).

9. The circuit (100) of claim 3, wherein the base of the further transistor (Q 12 IN) is shorted to the base of the first input transistor (Q 11 IN) by a shorting link (added link) that removes charge stored in the base of the second output transistor (Q 12 N) of the further Darlington transistor (Q 12 IN, Q 12 OUT).

10. The circuit (100) of claim 3, wherein the charge of the first output transistor (Q 11 OUT) is removed to cause the first output transistor (Q 11 OUT) to turn "OFF" when the second output transistor (Q 12 OUT) of the further Darlington transistor (Q 12 IN, Q 12 OUT) is turning on.

11. The circuit (100) of claim 9, wherein the shorting link (added link) discharges the first output transistor (Q 11 OUT) when the first output transistor (Q 11 OUT) is turning off.

12. The circuit (100) of claim 9, wherein the shorting link (added link) discharges the second output transistor (Q 12 OUT) when the second output transistor (Q12 OUT) is turning off.

## Patentansprüche

1. Eine Schaltung (100), beinhaltend:
einen Darlington-Transistor (Q11 IN, Q11 OUT) enthaltend:
einen ersten Eingangs-Transistor (Q 11 IN), der eine Basis hat, die mit einem Eingang (Input) verbunden ist;
einen ersten Ausgangs-Transistor (Q11 OUT), der eine Basis hat, die mit dem Emitter
des ersten Eingangs-Transistors (Q 11 IN) verbunden ist; und
einen weiteren Transistor (Q12 IN), der komplementär zum dem ersten Eingangs-Transistor (Q 11 IN) ist, **gekennzeichnet durch** das Vorhandensein einer Basis, die kurzgeschlossen ist zur Basis des ersten Eingangs-Transistors (Q11 IN), und eines Emitters, der kurzgeschlossen ist zur Basis des ersten Ausgangs-Transistors (Q 11 OUT).

2. Die Schaltung (100) nach Anspruch 1, in der der weitere Transistor (Q 12 IN) von einem Typ ist, der komplementär zu dem ersten Ausgangs Transistor (Q 11 OUT) des Darlington-Transistors (Q11 IN, Q11 OUT) ist.

3. Die Schaltung (100) nach Anspruch 1, in der der weitere Transistor (Q 12 IN) ein Eingangs-Transistor (Q 12 IN) eines weiteren Darlington-Transistors (Q 12 IN, Q12 OUT) ist.

4. Die Schaltung (100) nach Anspruch 3, in der der weitere Darlington Transistor (Q 12 IN, Q 12 OUT) einen zweiten Ausgangs-Transistor (Q 12 OUT) enthält, der eine Emitter hat, der mit dem Emitter des ersten Ausgangs-Transistors (Q 11 OUT) verbunden ist.

5. Die Schaltung (100) nach Anspruch 1, in der der weitere Transistor (Q 12 IN) mit Masse verbunden ist.

6. Die Schaltung (100) nach Anspruch 1, in der der weitere Transistor (Q 12 IN) mit einer Spannungsversorgung (Vcc) verbunden ist.

7. Die Schaltung (100) nach Anspruch 1, in der der erste Eingangs-Transistor (Q 11 IN) einen Kollektor hat, der mit der Spannungsversorgung (Vcc) verbunden ist, und in der der weitere Transistor (Q12 IN) einen Kollektor hat, der mit Masse verbunden ist.

8. Die Schaltung (100) nach Anspruch 1, wobei die Basis des weiteren Transistors (Q 12 IN) mit der Basis des ersten Eingangs-Transistors (Q 11 N) durch eine kurzschließende Verbindung (added link) kurzgeschlossen ist, die Ladung entfernt, die in der Basis des ersten Eingangs Transistors (Q 11 N) gespeichert ist.

9. Die Schaltung (100) nach Anspruch 3, wobei die Basis des weiteren Transistors (Q 12 IN) mit der Basis des ersten Eingangs-Transistors (Q 11 IN) durch eine kurzschließende Verbindung (added link) kurzgeschlossen ist, die Ladung entfernt, die in der Basis des zweiten Ausgangs-Transistors (Q 12 OUT) des weiteren Darlington-transistors (Q 12 IN, Q 12 OUT) gespeichert ist.

10. Die Schaltung (100) nach Anspruch 3, wobei die Ladung des ersten Ausgangs-Transistors (Q 11 OUT) entfernt wird um den ersten Ausgangs-Transistor (Q 11 OUT) zum Ausschalten zu veranlassen, wenn sich der zweite Ausgangs-Transistor (Q 12 OUT) des weiteren Darlington-Transistors (Q 12 IN, Q 12 OUT) eingeschaltet.

11. Die Schaltung (100) nach Anspruch 9, wobei die kurzschließende Verbindung (added link) den ersten Ausgangs-Transistor (Q 11 OUT) entlädt, wenn der erste Ausgangs-Transistor (Q 11 OUT) sich ausschaltet.

12. Die Schaltung (100) nach Anspruch 9, wobei die kurzschließende Verbindung (added link) den zweiten Ausgangs-Transistor (Q 12 OUT) entlädt, wenn der zweite Ausgangs-Transistor (Q12 OUT) sich ausschaltet.

## Revendications

1. Circuit (100) comprenant :
un transistor Darlington (Q11 IN, Q11 OUT) qui contient :
un premier transistor d'entrée (Q11 IN) doté d'une base connectée à une entrée (Input) ;
un premier transistor de sortie (Q11 OUT) doté d'une base connectée à l'émetteur du premier transistor d'entrée (Q11 IN) ; et
un transistor supplémentaire (Q12 IN) complémentaire du premier transistor d'entrée (Q11 IN)
**caractérisé par**
**le fait qu'**il est doté d'une base en contact direct avec la base du premier transistor d'entrée (Q11 IN) et
un émetteur en contact direct avec la base du premier transistor de sortie (Q11 OUT).

2. Circuit (100) selon la revendication 1, dans lequel le transistor supplémentaire (Q12 IN) est d'un type complémentaire du premier transistor de sortie (Q11 OUT) du transistor Darlington (Q11 IN, Q11 OUT).

3. Circuit (100) selon la revendication 1, dans lequel le transistor supplémentaire (Q12 IN) est un transistor d'entrée (Q12 IN) d'un transistor Darlington supplémentaire (Q12 IN, Q12 OUT).

4. Circuit (100) selon la revendication 3, dans lequel le transistor Darlington supplémentaire (Q12 IN, Q12 OUT) contient un deuxième transistor de sortie (Q12 OUT) doté d'un émetteur connecté à un émetteur du premier transistor de sortie (Q11 OUT).

5. Circuit (100) selon la revendication 1, dans lequel le transistor supplémentaire (Q12 IN) est connecté à la masse.

6. Circuit (100) selon la revendication 1, dans lequel le transistor supplémentaire (Q12 IN) est connecté à une alimentation (Vcc).

7. Circuit (100) selon la revendication 1, dans lequel le premier transistor d'entrée (Q11 IN) est doté d'un collecteur connecté à l'alimentation (Vcc) et le transistor supplémentaire (Q12 IN) est doté d'un collecteur connecté à la masse.

8. Circuit (100) selon la revendication 1, dans lequel la base du transistor supplémentaire (Q12 IN) est en contact direct avec la base du premier transistor d'entrée (Q11 IN) par une liaison de contact direct (added link) qui supprime la charge stockée dans la base du premier transistor d'entrée (Q11 IN).

9. Circuit (100) selon la revendication 3, dans lequel la base du transistor supplémentaire (Q12 IN) est en contact direct avec la base du premier transistor d'entrée (Q11 IN) par une liaison de contact direct (added link) qui supprime la charge stockée dans la base du deuxième transistor de sortie (Q12 OUT) du transistor Darlington supplémentaire (Q12 IN, Q12 OUT).

10. Circuit (100) selon la revendication 3, dans lequel la charge du premier transistor de sortie (Q11 OUT) est supprimée pour provoquer le blocage du premier transistor de sortie (Q11 OUT) lorsque le deuxième transistor de sortie (Q12 OUT) du transistor Darlington supplémentaire (Q12 IN, Q12 OUT) devient passant.

11. Circuit (100) selon la revendication 9, dans lequel la liaison de contact direct (added link) décharge le premier transistor de sortie (Q11 OUT) lorsque le premier transistor de sortie (Q11 OUT) devient bloqué.

12. Circuit (100) selon la revendication 9, dans lequel la liaison de contact direct (added link) décharge le deuxième transistor de sortie (Q12 OUT) lorsque le deuxième transistor de sortie (Q12 OUT) devient bloqué.
